**Europäisches Patentamt**

(19) **European Patent Office**

**Office européen des brevets**

(11) Veröffentlichungsnummer: **0 236 849**
**B1**

(12) # EUROPÄISCHE PATENTSCHRIFT

(45) Veröffentlichungstag der Patentschrift:
14.11.90

(21) Anmeldenummer: **87102665.4**

(22) Anmeldetag: **25.02.87**

(51) Int. Cl.⁵: **A61M 16/00**

(54) Vorrichtung zur Steuerung von Atemgas in einem Narkose.- bzw. Beatmungsgerät.

(30) Priorität: **06.03.86 DE 3607320**

(43) Veröffentlichungstag der Anmeldung:
**16.09.87 Patentblatt 87/38**

(45) Bekanntmachung des Hinweises auf die Patenterteilung:
**14.11.90 Patentblatt 90/46**

(84) Benannte Vertragsstaaten:
**DE FR GB IT SE**

(56) Entgegenhaltungen:
**DE-A- 2 945 472**
**US-A- 3 077 191**
**US-A- 4 381 002**

(73) Patentinhaber: **Drägerwerk Aktiengesellschaft,**
**Moislinger Allee 53-55, D-2400 Lübeck 1(DE)**

(72) Erfinder: **Kleinschmidt, Lothar, Dipl.-Ing., Lübecker**
**Strasse 32, D-2401 Krummesse(DE)**
Erfinder: **Waschmann, Michael, Dr., Rathenaustrasse 25,**
**D-2400 Lübeck(DE)**
Erfinder: **Wallroth, Carl-Friedrich, Dr.Dipl.-Ing.,**
**Stresemannstrasse 1, D-2400 Lübeck(DE)**

**Beschreibung**

Die Erfindung betrifft eine Vorrichtung zur Steuerung von Atemgas in einem Narkose- bzw. Beatmungsgerät, welche eine Vielzahl von Steuerelementen enthält, die je einen Steuerraum mit Steuerleitungen und einen Atemgasraum mit Atemgasleitungen besitzen, wobei der Steuerraum und der Atemgasraum durch ein Schließelement voneinander getrennt sind.

Aus der DE-OS 29 45 472 ist ein pneumatisches Steuersystem für ein Narkosebeatmungsgerät bekannt geworden, bei welchem über ein Mehrfachventil eine Umschaltung von automatischer Beatmung zur Handbeatmung und umgekehrt durchgeführt werden kann. Dazu werden die beiden Ventile wechselweise über eine entsprechende Druckbeaufschlagung des Steuerraums des einen Ventils geschaltet, wobei die beiden Membrandichtungen der Ventile über einen gemeinsamen Steg miteinander verbunden sind. Ist das eine Ventil geöffnet, ist das andere Ventil geschlossen und umgekehrt. Im Druckraum des zweiten Ventils befindet sich eine Druckfeder, welche die betreffende Membran geschlossen hält, solange im Druckraum des ersten Ventils kein entsprechender Druck vorliegt. Eine derartige Ventilkombination besteht aus einer Vielzahl von Einzelteilen, welche einen komplizierten Aufbau bedingen und bei einem notwendigen Reinigen oder Desinfizieren der mit dem Atemgas in Berührung kommenden Einzelteile ein aufwendiges Zerlegen und Zusammenbauen erforderlich machen.

Bei einem weiteren bekannten Beatmungsgerät nach DE-OS 33 03 998 ist zwar eine Trennung zwischen elektronisch arbeitendem Steuerteil und atemgasführenden Baugruppen angestrebt, jedoch sind auch hier die vielfältigen benötigten Ventile als Einzelgruppen aufgebaut und müssen jede für sich genommen zu Reinigungszwecken in ihre vielen Bestandteile zerlegt und anschließend wieder zusammengesetzt werden.

Die vorliegende Erfindung geht somit von der Aufgabenstellung aus, eine Vorrichtung zur Steuerung von Atemgas in einem Beatmungsgerät der genannten Art derart zu verbessern, daß bei einer Vielzahl von Steuerelementen deren Aufbau es ermöglicht, durch Zerlegen von wenigen Bauteilen die Gesamtheit der Steuerelemente zu Reinigungs- und Desinfektionszwecken frei zugänglich zu machen.

Die Aufgabe wird dadurch gelöst, daß die Steuerräume zu einem Steuerblock und die Atemgasräume zu einem Atemgasblock derart zusammengefaßt sind, daß die beiden Blöcke mittels einer gemeinsamen Dichtplatte mit Schließelementen zu einer Einheit koppelbar sind, in der die Steuerräume und die Atemgasräume getrennt und einander zugeordnet sind.

Die wesentlichen Vorteile der Erfindung liegen darin, daß durch den modulartigen Zusammenbau der einzelnen Blöcke eine besonders günstige Anordnung der Atemgasleitungen vorgenommen werden kann, so daß deren großlumigen Querschnitte einer Reinigung und Desinfektion leicht zugänglich sind. Außerdem wird die notwendige Zahl an Bauteilen, in welche die Vorrichtung zu Reinigungszwecken zerlegt werden muß, auf ein Mindestmaß beschränkt. Die Reinigung kann sich auf die Dichtplatte und die atemgasführenden Leitungen beschränken, und zwar unabhängig davon, wieviele Steuerräume oder Atemgasräume in den jeweiligen Blöcken zusammengefaßt sind. So ist es beispielsweise lediglich notwendig, die beiden Blöcke durch eine Schnellspannvorrichtung zu verbinden, wobei eine für alle Steuerelemente gemeinsame Dichtplatte mit Schließelementen zwischen ihnen eingespannt wird.

Ein weiterer Vorteil besteht darin, daß verschiedene Blöcke modulartig zusammengefaßt werden können, wobei die Mündungen von Steuerleitungen und Atemgasleitungen an den Außenflächen der Blöcke in geeigneter Weise als Schnittstelle verbunden werden.

Zweckmäßigerweise kann vorgesehen sein, daß die Wände der Atemgasleitungen zumindest teilweise von einem gemeinsamen, abnehmbaren Wandteil des Atemgasblocks gebildet sind. Dadurch kann erreicht werden, daß nach Abnehmen des Wandteils die Atemgasleitungen in ihrer vollen Länge zugänglich werden, um die Wirksamkeit einer Reinigung und Desinfektion noch weiter zu erhöhen.

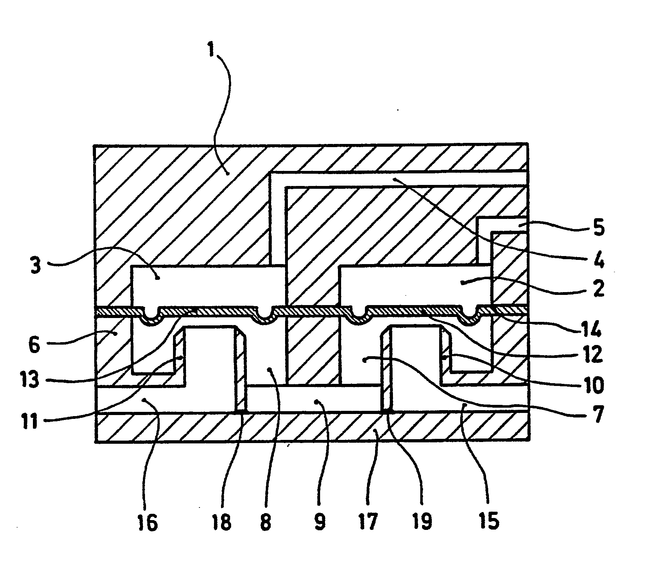

Ein Ausführungsbeispiel der Erfindung ist anhand der Zeichnung schematisch dargestellt und wird im folgenden näher erläutert.

Die einzige Figur zeigt im Schnitt eine Vorrichtung zur Steuerung von Atemgas und enthält einen Steuerblock (1), in welchem sich zwei Steuerräume (2) und (3) befinden. In diese Steuerräume (2) und (3) münden Steuerleitungen (4) und (5). Ein Atemgasblock (6) enthält zwei Atemgasräume (7) und (8), welche über eine Abströmleitung (9) miteinander verbunden sind. Jeder Atemgasraum (7) und (8) besitzt einen Dichtungskrater (10) und (11), gegen die je ein Schließelement (12) und (13) bewegbar angeordnet ist. Die Schließelemente (12) und (13) sind zu einer gemeinsamen Dichtplatte (14) miteinander verbunden, welche zwischen Steuerblock (1) und Atemgasblock (6) z.B. mit Hilfe nicht dargestellter, an den Außenflächen der Blöcke (1) und (6) angreifender Spannverschlüsse eingespannt ist. In der dargestellten Ausführungsform sind beide Ventile (10,12) und (11,13) in geöffnetem Zustand dargestellt, so daß Atemgas von den Einströmleitungen (15,16) durch beide Atemgasräume (7) und (8) in die Abströmleitung (9) gelangen kann. Je nach Druckbeaufschlagung der Steuerräume (2) und (3) über die ihnen zugeordneten Steuerleitungen (4) und (5) können die Schließelemente (12) und (13) gegen die Dichtungskrater (10) und (11) gedrückt werden und so eine Unterbrechung der Atemgasführung bewirken. Das den atemgasführenden Leitungen zugekehrte Wandteil (17) ist an den Trennfugen (18) und (19) abnehmbar, so daß von dieser Seite her ein freier Zugang zu den atemgasführenden Leitungen möglich ist.

In der dargestellten Form werden die Steuerräume (2) und (3) pneumatisch über die Steuerleitungen (4) und (5) mit Druck beaufschlagt. In gleicher Weise können aber auch mechanisch oder über einen geeigneten elektrischen Antrieb bewegbare Bauteile an die Schließelemente (12) und (13) angreifen, um diese gegenüber den Dichtungskratern (10) und (11) bewegbar zu gestalten.

## Patentansprüche

1. Vorrichtung zur Steuerung von Atemgas in einem Narkose- bzw. Beatmungsgerät, welche eine Vielzahl von Steuerelementen enthält, die je einen Steuerraum (2, 3) mit Steuerleitungen (4, 5) und einen Atemgasraum (7, 8) mit Atemgasleitungen (9, 15, 16) besitzen, wobei der Steuerraum und der Atemgasraum durch ein Schließelement (12, 13) voneinander getrennt sind, dadurch gekennzeichnet, daß die Steuerräume (2, 3) zu einem Steuerblock (1) und die Atemgasräume (7, 8) zu einem Atemgasblock (6) derart zusammengefaßt sind, daß die beiden Blöcke (1, 6) mittels einer gemeinsamen Dichtplatte (14) mit Schließelementen (12, 13) zu einer Einheit koppelbar sind, in der die Steuerräume (2, 3) und die Atemgasräume (7, 8) getrennt und einander zugeordnet sind.

2. Vorrichtung nach Anspruch 1, dadurch gekennzeichnet, daß die Wände der Atemgasleitungen (9, 15, 16) zumindest teilweise von einem gemeinsamen, abnehmbaren Wandteil (17) des Atemgasblocks (6) gebildet sind.

## Claims

1. Arrangement for the control of respiratory gas in an anaesthesia or respiratory apparatus, which arrangement contains a plurality of control elements, which have, respectively, a control chamber (2, 3) with control lines (4, 5) and a respiratory gas chamber (7, 8) with respiratory gas lines (9, 15, 16), the control chamber and the respiratory gas chamber being separated from each other by means of a closing element (12, 13), characterised in that the control chambers (2, 3) are combined to form a control block (1) and the respiratory gas chambers (7, 8) are combined to form a respiratory gas block (6) in such a manner that the two blocks (1, 6) can be coupled by means of a common sealing plate (14) with closing elements (12, 13) to form a unit in which the control chambers (2, 3) and the respiratory gas chambers (7, 8) are separated and coordinated with each other.

2. Arrangement according to claim 1, characterised in that the walls of the respiratory gas lines (9, 15, 16) are formed at least in part by a common, removable wall portion (17) of the respiratory gas block (6).

## Revendications

1. Dispositif pour la commande de gaz respiratoire d'un respirateur d'anesthésie ou d'un respirateur artificiel, comportant une pluralité d'éléments de commande qui possèdent chacun une chambre de commande (2, 3) avec des conduits de commande (4, 5) et une chambre de gaz respiratoire (7, 8) avec des conduits de gaz respiratoire (9, 15, 16), la chambre de commande et la chambre de gaz respiratoire étant séparées l'une de l'autre par un élément de fermeture (12, 13), caractérisé en ce que les chambres de commande (2, 3) sont réunies en un bloc de commande (1) et les chambres de gaz respiratoire (7, 8) en un bloc de gaz respiratoire (6), de manière que les deux blocs (1, 6) puissent être accouplés, au moyen d'une plaque d'étanchéité (14), commune, avec éléments de fermeture (12, 13), pour former une unité dans laquelle les chambres de commande (2, 3) et les chambres de gaz respiratoire (7, 8) sont séparées et associées l'une à l'autre.

2. Dispositif selon la revendication 1, caractérisé en ce que les parois des conduits de gaz respiratoire (9, 15, 16) sont formées, au moins partiellement, par une partie de paroi (17) commune, amovible, du bloc de gaz respiratoire.